(19) Europäisches Patentamt
European Patent Office
Office européen des brevets

(11) EP 1 944 014 A1

(12) **DEMANDE DE BREVET EUROPEEN**

(43) Date de publication:
**16.07.2008 Bulletin 2008/29**

(51) Int Cl.:
***A61K 8/86*** *(2006.01)* ***A61K 8/87*** *(2006.01)*
***A61Q 5/06*** *(2006.01)*

(21) Numéro de dépôt: **08300018.2**

(22) Date de dépôt: **09.01.2008**

(84) Etats contractants désignés:
**AT BE BG CH CY CZ DE DK EE ES FI FR GB GR HR HU IE IS IT LI LT LU LV MC MT NL NO PL PT RO SE SI SK TR**
Etats d'extension désignés:
**AL BA MK RS**

(30) Priorité: **12.01.2007 FR 0752653**

(71) Demandeur: **L'Oreal**
**75008 Paris (FR)**

(72) Inventeur: **Cothias, Pascale**
**78180 Montigny-Le-Bretonneux (FR)**

(74) Mandataire: **Catherine, Alain et al**
**Cabinet Harlé & Phélip**
**7, rue de Madrid**
**75008 Paris (FR)**

(54) **Composition cosmétique comprenant un polyuréthane cationique et un ester de polyéthylèneglycol et application au coiffage**

(57) L'invention concerne une composition cosmétique comprenant, dans un milieu aqueux cosmétiquement acceptable :
(i) au moins un polyuréthane cationique comportant au moins un motif non ionique dérivé d'un homopolymère ou d'un copolymère d'oléfine, et
(ii) au moins un ester de polyéthylène glycol.

EP 1 944 014 A1

## Description

La présente invention concerne de nouvelles compositions cosmétiques, comprenant l'association d'au moins un polyuréthane cationique comportant des motifs non ioniques dérivés d'au moins un homo- ou copolymère d'oléfine, et au moins un ester de polyéthylylène glycol.

La formation de dépôts et de films à propriétés élastiques fait depuis toujours l'objet d'importantes recherches en cosmétique. En effet, la plupart des zones du corps humain susceptibles de recevoir des dépôts cosmétiques, telles que la peau, les lèvres, les cheveux, les cils et les ongles sont soumises à des déformations et contraintes mécaniques importantes. Les films et dépôts cosmétiques doivent pouvoir résister à ces contraintes et suivre ces déformations sans se casser.

L'utilisation de polyuréthanes en cosmétique est connue depuis longtemps et est décrite par exemple dans les brevets WO94/13724 et EP0619111.

Les polyuréthanes décrits dans ces documents forment des films cassants inacceptables pour une application cosmétique.

Il existe certes d'autres polymères physiologiquement acceptables comme par exemple les polymères acryliques, mais ces polymères forment généralement des dépôts très collants, ce qui représente un inconvénient dans la plupart des applications cosmétiques.

L'utilisation des polyuréthanes cationiques à caractère élastique dans des compositions cosmétiques, notamment des compositions de coiffage, est connue.

Ainsi la demande de brevet français FR 2 815 350 décrit des polyuréthanes cationiques à caractère élastique et leur utilisation pour la formulation de laques et de compositions de coiffage améliorant la souplesse de la coiffure, c'est-à-dire permettant d'obtenir une tenue des cheveux plus naturelle que celle obtenue avec des polymères fixants usuels.

La demande de brevet français FR-2 833 960 décrit des compositions cosmétiques de coiffage, et en particulier des compositions de coiffage à rincer, notamment des shampoings coiffants, comportant un polyuréthane cationique ou amphotère auto-adhésif.

La Demanderesse a trouvé que l'utilisation d'un polyuréthane cationique comportant des motifs dérivés d'un homo- et/ou copolymère d'oléfine dans une composition capillaire apporte une excellente tenue dans le temps, en l'absence de sollicitations mécaniques ou d'eau ou d'humidité, mais avec des propriétés dégradées en présence de ces stimuli.

De manière surprenante, la demanderesse a découvert que des compositions cosmétiques comprenant ces polymères et contenant un ester de polyalkylèneglycol, permettent d'obtenir des coiffures plus résistantes aux sollicitations mécaniques, à l'eau et à l'humidité, par rapport aux formulations décrites ci-dessus. Le maintien est également plus naturel.

La présente invention a par conséquent pour objet une composition cosmétique, comprenant, dans un milieu aqueux cosmétiquement acceptable :

(i) au moins un polyuréthane cationique comportant au moins un motif non ionique dérivé d'un homo- et/ou copolymère d'oléfine, et
(ii) au moins un ester de polyéthylène glycol.

Les polyuréthanes cationiques préférés convenant pour l'invention contiennent :

(a) des motifs cationiques dérivés d'au moins un composé, de préférence une amine tertiaire ou quaternaire, présentant au moins deux fonctions réactives à hydrogène labile,
(b) des motifs non ioniques dérivés de polymères non ioniques portant à leurs extrémités des fonctions réactives à hydrogène labile, au moins un des motifs (b), de préférence au moins 50% en poids des motifs (b) et mieux la totalité des motifs (b) étant un motif ou des motifs (b1) dérivant d'un homo- ou copolymère d'oléfine portant à ses extrémités des fonctions réactives à hydrogène labile, et
(c) des motifs dérivés d'au moins un diisocyanate.

Par motif cationique, on entend au sens de la présente invention tout motif qui, soit par sa nature chimique propre, soit en fonction du milieu et/ou du pH dans lequel il se trouve, sera sous forme cationique.

On entend par fonctions réactives à hydrogène labile des fonctions capables, après départ d'un atome d'hydrogène, de former des liaisons covalentes avec les fonctions isocyanate des composés formant les motifs (c). On peut citer à titre d'exemple de telles fonctions les groupes hydroxyle, amine primaire ($-NH_2$) ou amine secondaire (-NHR), ou encore les groupes thiol (-SH).

La polycondensation de composés portant ces fonctions réactives à hydrogène labile avec des diisocayanates

donne, selon la nature des fonctions réactives portant l'hydrogène labile (-OH, $-NH_2$, -NHR ou -SH), respectivement des polyuréthanes, des polyurées ou des polythio-uréthanes. Tous ces polymères sont regroupés dans la présente demande, par souci de simplification, sous le terme de polyuréthanes. De préférence, les polymères de l'invention sont de vrais polyuréthanes.

Lorsque les amines tertiaires ou quaternaires formant les motifs (a) portent plus de deux fonctions à hydrogène labile les polyuréthanes obtenus présentent une structure ramifiée.

Dans un mode de réalisation préféré des polyuréthanes de la présente invention, les amines tertiaires ou quaternaires formant les motifs cationiques (a) ne présentent que deux fonctions réactives à hydrogène labile et les polyuréthanes obtenus par polycondensation ont par conséquent une structure essentiellement linéaire.

Il est bien entendu également possible d'utiliser un mélange d'amines difonctionnelles contenant une faible proportion d'amines portant plus de deux fonctions réactives à hydrogène labile.

Les amines tertiaires ou quaternaires formant les motifs cationiques (a) sont de préférence choisies parmi les composés correspondant à l'une des formules suivantes :

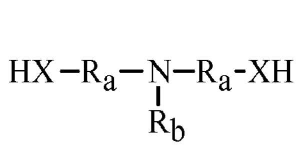
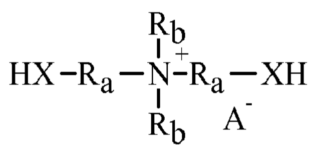

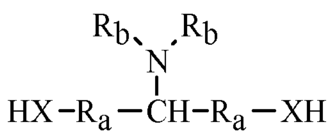
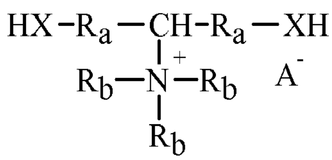

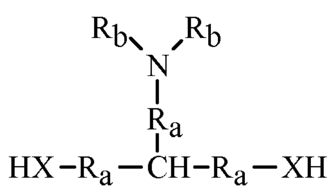
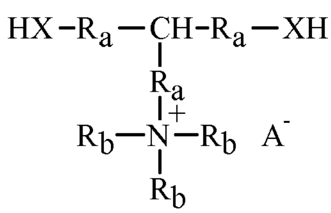

dans lesquelles

chaque $R_a$ représente indépendamment un groupe alkylène en $C_{1-6}$, linéaire ou ramifié, cycloalkylène en $C_{3-6}$ ou arylène, tous pouvant être substitués par un ou plusieurs atomes d'halogène et comporter un ou plusieurs hétéroatomes choisis parmi O, N, P et S,

chaque $R_b$ représente indépendamment un groupe alkyle en $C_{1-6}$, cycloalkyle en $C_{3-6}$ ou aryle, tous pouvant être substitués par un ou plusieurs atomes d'halogène et comporter un ou plusieurs hétéroatomes choisis parmi O, N, P et S,

chaque X représente indépendamment un atome d'oxygène ou de soufre ou un groupe NH ou $NR_c$, où $R_c$ représente un groupe alkyle en $C_{1-6}$, et

$A^-$ représente un contre-ion physiologiquement acceptable.

On peut citer à titre d'amines tertiaires particulièrement préférées pour l'obtention des polyuréthanes cationiques, la N-méthyldiéthanolamine et la N-tert-butyldiéthanolamine.

Les amines tertiaires et quaternaires formant les motifs cationiques (a) des polyuréthanes de la présente invention peuvent également être des polymères à fonction(s) amine tertiaire et/ou quaternaire, portant à leurs extrémités des fonctions réactives à hydrogène labile. La masse molaire moyenne en poids de ces polymères à fonctions amine tertiaire et/ou quaternaire est de préférence comprise entre 400 et 10 000.

On peut citer à titre d'exemples de tels polymères à fonctions amine appropriés les polyesters issus de la polycondensation de la N-méthyldiéthanolamine et de l'acide adipique.

Lorsque les amines formant les motifs cationiques (a) sont des composés à fonction(s) amine tertiaire, une partie ou la totalité de ces fonctions amine doit être neutralisée par un agent de neutralisation approprié choisi parmi les acides organiques ou minéraux physiologiquement acceptables. On peut citer à titre d'exemple d'acides préférés l'acide chlorhydrique ou l'acide acétique.

Le deuxième type de motifs formant les polyuréthanes préférés de la présente invention sont des motifs macromoléculaires, appelés motifs (b), dérivés de polymères non ioniques portant à leurs extrémités des fonctions réactives à hydrogène labile et ayant de préférence une température detransition vitreuse (Tg), mesurée par analyse enthalpique différentielle, inférieure à 10°C.

Selon l'invention, au moins un de ces motifs (b1) dérive d'un homo- ou copolymère d'oléfine.

Les propriétés viscoélastiques des polyuréthanes sont particulièrement avantageuses lorsque les motifs (b) sont dérivés de polymères ayant une température de transition vitreuse inférieure à 0°C et mieux encore inférieure à -10°C.

Ces polymères ont de préférence une masse molaire moyenne en poids comprise entre 400 et 10 000 et plus particulièrement entre 1000 et 5000.

Les polymères non ioniques susceptibles de former les motifs non ioniques (b2) différents des motifs non-ioniques (b1) dérivant des homo- et copolymères d'oléfine, peuvent être choisis parmi les polyéthers, les polyesters, les polysiloxanes, les polycarbonates et les polymères fluorés.

De préférence, les polymères susceptibles de former les motifs non-ioniques (b) sont choisis uniquement parmi les homo- et copolymères d'oléfine.

Parmi les polymères d'oléfine terminés par des groupes réactifs à hydrogène labile convenant pour la présente invention on peut citer les homopolymères et les copolymères statistiques ou blocs d'éthylène, de propylène, 1-butylène, 2-butylène, isobutylène, 1,2-butadiène, 1,4-butadiène et d'isoprène.

Les homo- et copolymères de butadiène et d'isoprène peuvent être partiellement ou totalement hydrogénés.

Les polymères préférés sont les copolymères d'éthylène/butylène, les polybutadiènes et les polybutadiènes hydrogénés porteurs de groupes réactifs à hydrogène labile à leurs extrémités et en particulier de groupes hydroxyles. Encore plus préférentiellement ces polymères sont des 1,2 et/ou 1,4 polybutadiènes.

De tels polymères sont disponibles commercialement par exemple sous la dénomination KRATON® L, en particulier KRATON® L 2203 (polybutadiènediol hydrogéné) auprès de la société KRATON polymers, KRASOL LBH® et LBHP®, notamment KRASOL LBHP® 2000 (polybutadiène diol) auprès de la société SARTOMER et GI® 3000 (copolymère éthylène/butylène) auprès de la société NISSO CHEMICAL.

Les diisocyanates formant les motifs (c) englobent les diisocyanates aliphatiques, alicycliques ou aromatiques.

Des diisocyanates préférés sont choisis parmi le méthylènediphényldiisocyanate, le méthylènecyclohexanediisocyanate, l'isophoronediisocyanate, le toluènediisocyanate, le natphtalènediisocyanate, le butanediisocyanate et l'hexyldiisocyanate. Ces diisocyanates peuvent bien entendu être utilisés seuls ou sous forme de mélange de deux ou plusieurs diisocyanates. Encore plus préférentiellement le diisocyanate est l'isophoronediisocyanate.

Comme indiqué ci-avant, les polyuréthanes cationiques de la présente invention peuvent contenir, en plus des motifs (a), (b1) et (c), une certaine fraction de motifs (b2) dérivés de composés, en général monomères, non ioniques contenant au moins deux fonctions à hydrogène labile et différents des composés conduisant aux motifs (b1).

Ces motifs (b2) sont dérivés en général de diols en $C_1$-$C_{12}$, par exemple de néopentylglycol, d'hexaéthylèneglycol, de 1,2-éthanediol, de 1,2-propanediol et de 1,3-propanediol ou d'aminoalcools, en $C_1$-$C_6$ par exemple l'aminoéthanol.

Les polyuréthanes cationiques de l'invention présentent de préférence un caractère élastique.

Dans un mode de réalisation particulier de l'invention, le polyuréthane cationique ne comprend pas de motifs en plus des motifs (a), (b) et (c). Un polyuréthane répondant à cette définition est le polyuréthane (A) décrit dans les exemples.

Dans un mode de réalisation alternatif, le polyuréthane cationique comprend des motifs en plus des motifs (a), (b) et (c). Un polyuréthane répondant à cette définition est le polyuréthane (B) décrit dans les

exemples.

Un paramètre physique caractérisant les propriétés visco-élastiques des polyuréthanes cationiques ci-dessus est leur recouvrance en traction. Cette recouvrance est déterminée par essai de fluage en traction consistant à étirer rapidement une éprouvette jusqu'à un taux d'allongement prédéterminé, puis à relâcher la contrainte et à mesurer la longueur de l'éprouvette.

L'essai de fluage utilisé pour la caractérisation des polyuréthanes cationiques à caractère élastique de la présente invention se déroule de la manière suivante :

On utilise, comme éprouvette, un film du polyuréthane ayant une épaisseur de 500 ± 50 mm, découpé en bandes de 80 mm x 15 mm. Ce film de copolymère est obtenu par séchage, à une température de 22 ± 2 °C et à une humidité relative de 50 ± 5 %, d'une solution ou dispersion à 3 % en poids dudit polyuréthane dans de l'eau et/ou de l'éthanol.

Chaque bande est fixée entre deux mors, distants de 50 ± 1 mm l'un de l'autre, et est étirée à une vitesse de 20 mm/minute (dans les conditions de température et d'humidité relative ci-dessus) jusqu'à un allongement de 50 % ($\varepsilon_{max}$), c'est-à-dire l'obtention d'une bande présentant jusqu'à 1,5 fois sa longueur initiale. On relâche alors la contrainte en imposant une vitesse de retour égale à la vitesse de traction, soit 20 mm/minute, et on mesure l'allongement de l'éprouvette (exprimé en % par rapport à la longueur initiale) immédiatement après retour à charge nulle ($\varepsilon_i$).

La recouvrance instantanée ($R_i$) est calculée à l'aide de la formule suivante :

$$R_i\ (\%) = ((\varepsilon_{max} - \varepsilon_i)/\ \varepsilon_{max})\ x\ 100$$

Les polyuréthanes cationiques à caractère élastique de la présente invention ont de préférence une recouvrance instantanée ($R_i$), mesurée dans les conditions indiquées ci-dessus, comprise entre 5 % et 95 %, en particulier comprise entre 20 % et 90 % et idéalement entre 35 et 85 %.

La température de transition vitreuse (Tg) des polymères non ioniques formant les motifs (b) et des polyuréthanes cationiques de la présente invention est mesurée par analyse enthalpique différentielle (DSC, *differential scanning calorimetry)* selon la norme ASTM D3418-97.

Les polyuréthanes cationiques à caractère élastique de la présente invention présentent de préférence au moins deux températures de transition vitreuse, dont une au moins est inférieure à 10°C, de préférence inférieure à 0°C et mieux encore inférieure à -10°C, et au moins une autre est supérieure ou égale à la température ambiante (20°C).

La recouvrance instantanée et par conséquent les propriétés viscoélastiques des polyuréthanes de la présente invention dépend des fractions des différents motifs monomères (a), (b1), (b2) et (c).

La fraction de motifs (a) doit être de préférence suffisante pour conférer aux polymères leur charge positive responsable de leur bonne affinité pour les substrats kératiniques. Les motifs (b) doivent de préférence représenter une fraction en poids suffisante pour que les polyuréthanes présentent de préférence au moins une température de transition vitreuse inférieure à 10 °C et ne forment pas des films cassants.

De manière préférée, les motifs (a) représentent de 0,1 à 90 %, de préférence de 1 à 30 %, mieux de 5 à 25% et idéalement de 5 à 10% en poids, les motifs (b1) de 10 à 99,9 %, de préférence de 20 à 99 % et mieux de 30 à 85% en poids et les motifs (b2) de 0 à 50 % en poids, de préférence de 0 à 30 % en poids par rapport au poids total des motifs du polyuréthane. De préférence encore les polyuréthanes de l'invention ne comportent pas de motif (b2).

De manière préférée les motifs (c) représentent de 1 à 60% mieux de 5 à 50% idéalement de 10 à 40% du poids total des motifs polyuréthane

Les motifs (c) sont de préférence présents en une quantité essentiellement stoechiométrique par rapport à la somme des motifs (a) et (b). En effet, l'obtention de polyuréthanes ayant des masses molaires importantes suppose un nombre de fonctions isocyanate pratiquement identique au nombre de fonction à hydrogène labile. L'homme du métier saura choisir un éventuel excès molaire de l'un ou de l'autre type de fonction pour ajuster la masse molaire à la valeur désirée.

La quantité de polyuréthane présente dans une composition cosmétique objet de l'invention, dépend bien entendu du type de composition et des propriétés recherchées et peut varier à l'intérieur d'une gamme très large, comprise généralement de préférence entre 0,01 et 40 % en poids, de préférence entre 0,05 et 20 %, idéalement entre 0,1 et 10% en poids, rapporté à la composition cosmétique finale.

De manière tout à fait préférée, l'ester de polyéthylène glycol est défini par la formule suivante :

$$X\text{-}(OCH_2CH_2)_mOY \qquad (1)$$

- m allant de 80 à 500 et
- X et Y représentant, indépendemment l'un de l'autre un atome d'hydrogène, ou un groupement acyle en $C_8$ à $C_{30}$ linéaire ou ramifié, saturé ou insaturé,

à condition que l'un au moins des groupements X et Y représente un groupement acyle.

De préférence, du polyéthylène glycol comprenant 80 à 500 groupes d'oxyde d'éthylène par molécule est utilisé pour obtenir l'ester de polyéthylène glycol de formule (I) ci-dessus.

A titre d'exmple de polyéthylène glycol préféré, on peut citer :

- le polyéthylène glycol PEG-90, comprenant un nombre moyen de groupements éthylène glycol de 90 et ayant une masse moléculaire moyenne théorique Mth d'environ 4000 $g.mol^{-1}$,
- le polyéthylène glycol PEG-100, comprenant un nombre moyen de groupements éthylène glycol de 100 et ayant une masse moléculaire moyenne théorique Mth d'environ 4400 $g.mol^{-1}$,
- le polyéthylène glycol PEG-135, comprenant un nombre moyen de groupements éthylène glycol de 135 et ayant une masse moléculaire moyenne théorique Mth d'environ 6000 $g.mol^{-1}$,
- le polyéthylène glycol PEG-150, comprenant un nombre moyen de groupements éthylène glycol de 150 et ayant une masse moléculaire moyenne théorique Mth d'environ 6600 $g.mol^{-1}$,
- le polyéthylène glycol PEG-180, comprenant un nombre moyen de groupements éthylène glycol de 180 et ayant une masse moléculaire moyenne théorique Mth d'environ 7900 $g.mol^{-1}$,
- le polyéthylène glycol PEG-200, comprenant un nombre moyen de groupements éthylène glycol de 200 et ayant une masse moléculaire moyenne théorique Mth d'environ 8800 $g.mol^{-1}$,
- le polyéthylène glycol PEG-240, comprenant un nombre moyen de groupements éthylène glycol de 240 et ayant une masse moléculaire moyenne théorique Mth d'environ 10600 $g.mol^{-1}$,
- le polyéthylène glycol PEG-350, comprenant un nombre moyen de groupements éthylène glycol de 350 et ayant une masse moléculaire moyenne théorique Mth d'environ 15400 $g.mol^{-1}$,
- le polyéthylène glycol PEG-454, comprenant un nombre moyen de groupements éthylène glycol de 454 et ayant une masse moléculaire moyenne théorique Mth d'environ 20 000 $g.mol^{-1}$,

La masse moléculaire moyenne théorique Mth d'un polyéthylène glycol étant calculée d'après l'équation (I) :

$$Mth = 18 + 44xm \qquad (I)$$

où m est tel que défini précédemment.

De préférence m va de 80 à 350 et encore plus préférentiellement de 100 à 300.

De préférence, le rapport (R) :

$$R = \text{Masse de la partie hydrophile } (OCH_2CH_2)_m / (\text{Masse de l'ester de polyéthylène glycol} - \text{masse de la partie hydrophile } (OCH_2CH_2)_m)$$

est compris entre 8 et 1000

A titre d'ester de polyéthylène glycol de formule (1) on peut citer les monoesters de polyéthylène glycol tels que les produits référencés dans le « International Cosmetic Ingredient Dictionary and Handbook » 9ème édition, 2002, sous les références PEG90 STEARATE, PEG100 STEARATE, PEG120 STEARATE, PEG150 STEARATE, PEG150 LAURATE, et PEG150 OLEATE.

Parmi les diesters de polyéthylène glycol, on peut citer les produits référencés dans le « International Cosmetic Ingredient Dictionary and Handbook » 9ème édition, 2002, sous les références PEG150 DILAURATE, PEG150 DIOLEATE, PEG120 DISTEARATE, PEG150 DISTEARATE, PEG175 DISTEARATE, et PEG250 DISTEARATE.

L'ester de polyéthylène glycol peut être un monoester ou un diester. De préférence, l'ester de polyéthylène glycol est un diester.

La quantité d'ester de polyéthylène glycol dans une composition cosmétique objet de l'invention, dépend bien entendu du type de composition et des propriétés recherchées et peut varier à l'intérieur d'une gamme très large, comprise généralement entre 0,01 et 50 % en poids, de préférence entre 0,1 et 20 %, encore plus préférentiellement entre 0,5 et 15% en poids, rapporté au poids de la composition cosmétique finale, étant entendu qu'un ou plusieurs types d'esters de polyéthylène glycol peuvent être incorporés à la composition cosmétique. Par exemple un monoester et un diester peuvent être incorporés à la composition cosmétique selon l'invention.

De préférence, le milieu cosmétiquement acceptable est aqueux.

La composition cosmétique selon l'invention peut comprendre en outre un solvant organique, de préférence dans une quantité comprise entre 0,05 et 40%, de manière tout à fait préférée, entre 1 et 20% en poids, par rapport au poids total de la composition.

Ce solvant organique peut être un alcool inférieur en C2 à C4, en particulier l'éthanol, un polyol tel que le propylène glycol ou le glycérol ou un éther de polyol.

Les compositions selon l'invention peuvent également contenir d'autres adjuvants cosmétiquement acceptables, tels que par exemple des agents tensioactifs, (autres qu'en ester de polyéthylèneglycol, certains esters de polyéthylèneglycol pouvant être des tensioactifs), des agents épaississants, des agents de pénétration, des parfums, des tampons, et divers adjuvants usuels comme des filtres UV, des cires, des silicones volatiles ou non, cycliques ou linéaires ou ramifiées, organomodifiées (notamment par des groupements amines) ou non, des conservateurs, des céramides, des pseudocéramides, des huiles végétales, minérales ou de synthèse, les vitamines ou provitamines comme le panthénol, des opacifiants, des agents réducteurs, des émulsionnants, des conservateurs, des charges, des filtres solaires, des protéines, des polymères fixants anioniques, non ioniques, cationiques ou amphotères, des agents hydratants, des émollients, des agents adoucissants, des agents anti-mousse, des agents antiperspirants, des agents anti-radicaux libres, des polymères fixants ou non, des bactéricides, des séquestrants, des antipelliculaires, des anti-oxydants, des agents alcalinisants, et tout autre additif classiquement utilisé dans les compositions cosmétiques destinées à être appliquées sur les cheveux.

Les agents tensioactifs, différents des esters de polyéthylèneglycol, utilisables dans la composition selon la présente invention peuvent être des tensioactifs anioniques, non ioniques, amphotères ou cationiques, ou des mélanges de ceux-ci.

Parmi les tensioactifs anioniques utilisables, seuls ou en mélanges, dans le cadre de la présente invention, on peut notamment citer les sels, et en particulier les sels de métaux alcalins tels que les sels de sodium, les sels d'ammonium, les sels d'amines, les sels d'aminoalcools ou les sels de métaux alcalino-terreux, par exemple, de magnésium, des composés suivants : les alkylsulfates, les alkyléthersulfates, les alkylamidoéthersulfates, les alkylarylpolyéthersulfates, les monoglycéride-sulfates ; les alkylsulfonates, les alkylamidesulfonates, les alkyl-arylsulfonates, les $\alpha$-oléfine-sulfonates, les paraffine-sulfonates ; les alkylsulfosuccinates, les alkyléthersulfosuccinates, les alkylamide-sulfosuccinates ; les alkylsulfoacétates ; les acylsarconisates ; et les acylglutamates, les groupes alkyle et acyle de tous ces composés comportant de 6 à 24 atomes de carbone et le groupe aryle désignant de préférence un groupe phényle ou benzyle.

On peut également utiliser dans le cadre de la présente invention les esters d'alkyle en $C_6$-$C_{24}$ et d'acides polyglycoside-carboxyliques tels que les glucoside-citrates d'alkyle, les polyglycoside-tartrates d'alkyle, et les polyglycoside-sulfosuccinates d'alkyle ; les alkylsulfosuccinamates, les acyliséthionates et les N-acyltaurates, le groupe alkyle ou acyle de tous ces composés comportant de 12 à 20 atomes de carbone. Parmi les tensioactifs anioniques encore utilisables, on peut également citer les acyllactylates dont le groupe acyle comporte de 8 à 20 atomes de carbone.

En outre, on peut encore citer les acides d'alkyl-D-galactoside uroniques et leurs sels ainsi que les acides alkyl ($C_6$-$C_{24}$)éther-carboxyliques polyoxyalkylénés, les acides alkyl($C_6$-$C_{24}$)aryl($C_6$-$C_{24}$)éther-carboxyliques polyoxyalkylénés, les acides alkyl($C_6$-$C_{24}$)amidoéther carboxyliques polyoxyalkylénés et leurs sels, en particulier ceux comportant de 2 à 50 groupements oxyde d'éthylène, et leurs mélanges.

Parmi les tensioactifs anioniques cités ci-dessus, on préfère utiliser selon l'invention les alkyl($C_6$-$C_{24}$)sulfates, les alkyl($C_6$-$C_{24}$)éthersulfates, les alkyl($C_6$-$C_{24}$)éthercarboxylates et leurs mélanges, par exemple le laurylsulfate d'ammonium, le laurylsulfate de sodium, le laurylsulfate de magnésium, le lauryléthersulfate de sodium, le lauryléthersulfate d'ammonium et le lauryléthersulfate de magnésium.

La composition selon la présente invention peut comprendre les agents tensioactifs anioniques en une quantité comprise de préférence entre 0,5 et 60% en poids, mieux encore entre 5 et 20% en poids par rapport au poids total de la composition.

Les agents tensioactifs non-ioniques que l'on peut utiliser dans le cadre de la présente invention, sont eux aussi, des composés bien connus en soi (voir notamment à cet égard "Handbook of Surfactants" par M.R. PORTER, éditions Blackie & Son (Glasgow and London), 1991, pp 116-178) Ils peuvent être notamment choisis parmi les alcools, les alpha-diols, les alkyl(C1-C20)phénols ou les acides gras polyéthoxylés, polypropoxylés ou polyglycérolés, ayant une chaîne grasse comportant par exemple de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et d'oxyde de propylène, les condensats d'oxyde d'éthylène et d'oxyde de propylène sur des alcools gras ; les amides gras polyéthoxylés ayant de préférence de 2 à 30 moles d'oxyde d'éthylène ; les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitane éthoxylés ayant de 2 à 30 moles d'oxyde d'éthylène ; les esters d'acides gras du saccharose, les esters d'acide gras du polyéthylèneglycol, les alkyl($C_6$-$C_{24}$)polyglucosides, les dérivés de N-alkyl($C_6$-$C_{24}$)glucamine, les oxydes d'amines tels que les oxydes d'alkyl($C_{10}$-$C_{14}$)amines ou les oxydes de N-acyl($C_{10}$-$C_{14}$)aminopropylmorpholine; et leurs mélanges.

Parmi les tensioactifs non-ioniques cités ci-dessus, on utilise de préférence les alkyl($C_6$-$C_{24}$)polyglycosides, en particulier le décylpolyglucoside.

Les agents tensioactifs amphotères, convenant dans la présente invention, peuvent être notamment des dérivés d'amines aliphatiques secondaires ou tertiaires, dans lesquels le groupe aliphatique est une chaîne linéaire ou ramifiée comportant 8 à 22 atomes de carbone et contenant, au moins un groupe anionique hydrosolubilisant tel que, par exemple, un groupe carboxylate, sulfonate, sulfate, phosphate ou phosphonate, on peut citer encore les alkyl($C_8$-$C_{20}$)bétaïnes,

les sulfobétaïnes, les alkyl($C_8$-$C_{20}$)amidoalkyl($C_6$-$C_8$)-bétaïnes ou les alkyl($C_8$-$C_{20}$)amidoalkyl($C_6$-$C_8$)Sulfobétaïnes ; et leurs mélanges.

Parmi les dérivés d'amines, on peut citer les produits commercialisés sous la dénomination MIRANOL®, tels que décrits dans les brevets US-2,528,378 et US-2,781,354 et classés dans le dictionnaire CTFA, 3ème édition, 1982, sous les dénominations Amphocarboxy-glycinate et Amphocarboxypropionate de structures respectives (1) et (2) :

$$R_2\text{-CONHCH}_2\text{CH}_2\text{-N}^+(R_3)(R_4)(\text{CH}_2\text{COO}^-) \qquad (1)$$

dans laquelle :

- $R_2$ désigne représente un groupe alkyle dérivé d'un acide $R_2$-COOH présent dans l'huile de coprah hydrolysée, un groupe heptyle, nonyle ou undécyle,
- $R_3$ représente un groupe bêta-hydroxyéthyle, et
- $R_4$ représente un groupe carboxyméthyle;

Et

$$R_2\text{-CONHCH}_2\text{CH}_2\text{-N(B)(C)} \qquad (2)$$

dans laquelle :

- B représente $-CH_2CH_2OX'$,
- C représente $-(CH_2)_z$ -Y', avec z = 1 ou 2,
- X' représente le groupement $-CH_2CH_2$-COOH ou un atome d'hydrogène,
- Y' représente -COOH ou le groupe $-CH_2$ - CHOH - $SO_3H$,
- $R_2$ représente le groupe alkyle d'un acide $R_{2'}$-COOH présent dans l'huile de coprah ou dans l'huile de lin hydrolysée, un groupe alkyle, notamment en $C_{17}$ et sa forme iso, un groupe $C_{17}$ insaturé.

Ces composés sont classés dans le dictionnaire CTFA, 5ème édition, 1993, sous les dénominations cocoamphodiacétate de disodium, lauroamphodiacétate de disodium, caprylamphodiacétate de disodium capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique.

A titre d'exemple on peut citer le cocoamphodiacétate commercialisé sous la dénomination commerciale MIRANOL® C2M concentré par la société RHODIA.

Parmi les tensioactifs amphothères, on utilise de préférence les alkyl($C_8$-$C_{20}$)bétaïnes telles que la cocobétaïne, les alkyl($C_8$-$C_{20}$)amidoalkyl($C_6$-$C_8$)bétaïnes telles que la cocamidobétaïne, les alkylamphodiacétates comme le cocoamphodiacétate disodique, et leurs mélanges.

La composition selon l'invention peut comprendre en outre un ou plusieurs tensioactifs cationiques bien connus en soi, tels que les sels d'amines grasses primaires, secondaires ou tertiaires, éventuellement polyoxyalkylénées, les sels d'ammonium quaternaire tels que les chlorures ou les bromures de tétraalkylammonium, d'alkylamidoalkyltrialkylammonium, de trialkylbenzylammonium, de trialkylhydroxyalkylammonium ou d'alkylpyridinium, les dérivés d'imidazoline ; ou les oxydes d'amines à caractère cationique.

Les agents tensioactifs non ioniques, amphotères et cationiques décrits ci-dessus peuvent être utilisés seuls ou en mélanges et leur quantité est comprise entre 0,1% et 30% en poids, de préférence entre 0,5% et 25% en poids et mieux encore entre 1% et 20% en poids par rapport au poids total de la composition.

Les agents gélifiants et/ou épaississants convenant pour les compositions de l'invention sont bien connus dans la technique et peuvent être choisis parmi les polymères et copolymères carboxy-vinyliques, les polymères et copolymères (alkyl)acryliques, les polymères et copolymères (alkyl)acrylamides, les poly(oxyalkylène)glycols, les esters de poly(oxyalkylène)glycols, les alginates, les biosaccharides, les polysaccharides tels que les dérivés de cellulose et d'amidon, les gommes naturelles telles que les gommes de xanthane, de guar, de caroube, les scléroglucanes, les dérivés de chitine et de chitosane, les carraghénanes, les argiles, et leur mélanges.

A titre d'exemple d'agents gélifiants, notamment en phase aqueuse, on peut citer le SEPIGEL® 305 commercialisé par la société SEPPIC, le FUCOGEL® 1000 PP commercialisé par la société SOLABIA, le SYNTHALEN® K commercialisé par la société 3VSA, le LUVISKOL® VA 64 P commercialisé par la société BASF, l'HOSTACERIN® AMPS commercialisé par la société CLARIANT, le PEMULEN® TR1 commercialisé par la société GOODRICH, le LUBRAGEL® MS commercialisé par la société GUARDIAN, le SATIAGEL® KSO commercialisé par DEGUSSA et le KELTROL® commercialisé par la société KELCO.

L'agent gélifiant représente en général de 0,05 à 15%, de préférence de 0,5 à 10% en poids de la composition.

Les silicones utilisables en tant qu'additifs dans les compositions cosmétiques de la présente invention, sont des silicones volatiles ou non volatiles, cycliques, linéaires ou ramifiées, modifiées ou non par des groupements organiques, ayant une viscosité de $5.10^{-6}$ à $2{,}5 m^2/s$ à 25°C et de préférence $1.10^{-5}$ à $1 m^2/s$.

Les silicones utilisables conformément à l'invention peuvent être solubles ou insolubles dans la composition et en particulier être des polyorganosiloxanes insolubles dans la composition de l'invention. Elles peuvent se présenter sous forme d'huiles, de cires, de résines ou de gommes.

Les organopolysiloxanes sont définis plus en détail dans l'ouvrage de Walter NOLL "Chemistry and Technology of Silicones" (1968), Academie Press. Elles peuvent être volatiles ou non volatiles.

Lorsqu'elles sont volatiles, les silicones sont plus particulièrement choisies parmi celles possédant un point d'ébullition compris entre 60°C et 260°C, et plus particulièrement encore parmi:

(i) les silicones cycliques comportant de 3 à 7, de préférence de 4 à 5 atomes de silicium. Il s'agit, par exemple, de l'octaméthylcyclotétrasiloxane commercialisé notamment sous le nom de VOLATILE SILICONE® 7207 par UNION CARBIDE ou SILBIONE® 70045 V2 par RHODIA, le décaméthylcyclopentasiloxane commercialisé sous le nom de VOLATILE SILICONE® 7158 par UNION CARBIDE, et SILBIONE® 70045 V5 par RHODIA, ainsi que leurs mélanges. On peut également citer les cyclocopolymères du type diméthylsiloxanes/ méthylalkylsiloxane, tel que la SILICONE VOLATILE® FZ 3109 commercialisée par la société UNION CARBIDE, de formule : avec

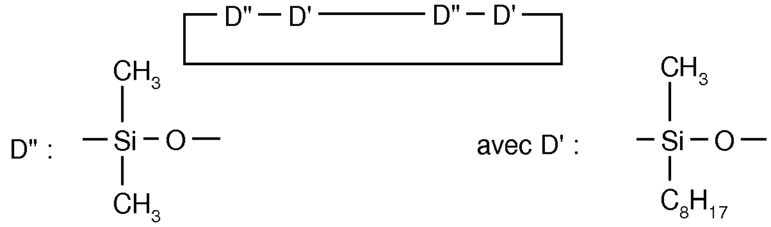

D" : avec D' :

On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bis-néopentane ;

(ii) les silicones volatiles linéaires ayant 2 à 9 atomes de silicium et présentant une viscosité inférieure ou égale à $5.10^{-6} m^2/s$ à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and Toiletries, Vol. 91, Jan. 76, P. 27-32 - TODD & BYERS "Volatile Silicone fluids for cosmetics".

On utilise de préférence des silicones non volatiles, et plus particulièrement des polyalkylsiloxanes, des polyarylsiloxanes, des polyalkylarylsiloxanes, des gommes et des résines de silicones, des polyorganosiloxanes modifiés par les groupements organofonctionnels ainsi que leurs mélanges.

Ces silicones sont plus particulièrement choisies parmi les polyalkylsiloxanes parmi lesquels on peut citer principalement les polydiméthylsiloxanes à groupements terminaux triméthylsilyl. La viscosité des silicones est mesurée à 25°C selon la norme ASTM 445 Appendice C.

Parmi ces polyalkylsiloxanes, on peut citer à titre non limitatif les produits commerciaux suivants :

- les huiles SILBIONE® des séries 47 et 70 047 ou les huiles MIRASIL® commercialisées par RHODIA telles que, par exemple l'huile 70 047 V 500 000 ;
- les huiles de la série MIRASIL® commercialisées par la société RHODIA ;
- les huiles de la série 200 de la société DOW CORNING telles que la DC200 ayant viscosité 60 000 $mm^2/s$ ;
- les huiles VISCASIL® de GENERAL ELECTRIC et certaines huiles des séries SF (SF 96, SF 18) de GENERAL ELECTRIC.

On peut également citer les polyméthylsiloxanes à groupements terminaux diméthylsilanol connus sous le nom de dimethiconol (CTFA), tels que les huiles de la série 48 de la société RHODIA.

Dans cette classe de polyalkylsiloxanes, on peut également citer les produits commercialisés sous les dénominations "ABIL WAX® 9800 et 9801" par la société GOLDSCHMIDT qui sont des polyalkyl ($C_1$-$C_{20}$) siloxanes.

Les polyalkylarylsiloxanes sont particulièrement choisis parmi les polydiméthyl/méthylphénylsiloxanes, les polydiméthyl/diphénylsiloxanes linéaires et/ou ramifiés de viscosité allant de $1.10^{-5}$ à $5.10^{-2} m^2/s$ à 25°C.

Parmi ces polyalkylarylsiloxanes on peut citer à titre d'exemple les produits commercialisés sous les dénominations suivantes :

- les huiles SILBIONE® de la série 70 641 de RHODIA;
- les huiles des séries RHODORSIL® 70 633 et 763 de RHODIA ;
- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000 ;
- certaines huiles des séries SF de GENERAL ELECTRIC telles que SF 1023, SF 1154, SF 1250, SF 1265.

Les gommes de silicone utilisables conformément à l'invention sont notamment des polyorganosiloxanes, ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles polyphénylméthylsiloxanes (PPMS), les isoparaffines, les polyisobutylènes, le chlorure de méthylène, le pentane, le dodécane, le tridécane ou leurs mélanges.
On peut citer plus particulièrement les produits suivants :

- polydiméthylsiloxane,
- les gommes polydiméthylsiloxane/méthylvinylsiloxane,
- polydiméthylsiloxane/diphénylsiloxane,
- polydiméthylsiloxane/phénylméthylsiloxane,
- polydiméthylsiloxane/diphénylsiloxane/méthylvinylsiloxane.

Des produits plus particulièrement utilisables conformément à l'invention sont des mélanges tels que :

- les mélanges formés à partir d'un polydiméthylsiloxane hydroxylé en bout de chaîne, ou diméthiconol (CTFA) et d'un polydiméthylsiloxane cyclique également appelé cyclométhicone (CTFA) tel que le produit Q2 1401 commercialisé par la société DOW CORNING ;
- les mélanges d'une gomme polydiméthylsiloxane et d'une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 $m^2/s$ et d'une huile SF 96 d'une viscosité de $5.10^{-6}m^2/s$. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les motifs : $R_2SiO_{2/2}$, $R_3SiO_{1/2}$, $RSiO_{3/2}$ et $SiO_{4/2}$
dans lesquelles R représente un groupement hydrocarboné possédant 1 à 16 atomes de carbone ou un groupement phényle. Parmi ces produits, ceux particulièrement préférés sont ceux dans lesquels R désigne un groupe alkyle inférieur en $C_1$-$C_4$, plus particulièrement méthyle ou un groupe phényle.
On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.
On peut également citer les résines du type triméthylsiloxysilicate commercialisées notamment sous les dénominations X22-4914, X21-5034 et X21-5037 par la société SHIN-ETSU.
Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.
Parmi les silicones organomodifiées, on peut citer les polyorganosiloxanes comportant :

- des groupements polyéthylèneoxy et/ou polypropylèneoxy comportant éventuellement des groupements alkyle en $C_6$-$C_{24}$ tels que les produits dénommés diméthicone copolyol commercialisé par la société DOW CORNING sous la dénomination DC 1248 ou les huiles SILWET® L 722, L 7500, L 77, L 711 de la société UNION CARBIDE et l'alkyl ($C_{12}$)-méthicone copolyol commercialisée par la société DOW CORNING sous la dénomination Q2 5200 ;
- des groupements aminés substitués ou non comme les produits commercialisés sous la dénomination GP 4 Silicone Fluid et GP 7100 par la société GENESEE ou les produits commercialisés sous les dénominations Q2 8220 et DOW

CORNING 929 ou 939 par la société DOW CORNING. Les groupements aminés substitués sont en particulier des groupements aminoalkyles en $C_1$-$C_4$ ;

- des groupements thiols, comme les produits commercialisés sous les dénominations GP 72A et GP 71 de GENESEE.
- des groupements alcoxylés, comme le produit commercialisé sous la dénomination "SILICONE COPOLYMER F-755" par SWS SILICONES et ABIL WAX® 2428, 2434 et 2440 par la société GOLDSCHMIDT.
- des groupements hydroxylés, comme les polyorganoxiloxanes à fonction hydroxyalkyle décrits dans la demande de brevet français FR-A- 8 516 334 ;
- des groupements alcoxyalkyle tels que, par exemple, les polyorganosiloxanes décrits dans le brevet US-A-4 957 732 ;
- des groupements anioniques du type carboxylique comme par exemple, dans le produits décrits dans le brevet EP 186 507 de la société CHISSO CORPORATION, ou de type alkyl-carboxyliques comme ceux présents dans le produit X-22-3701 E de la société SHIN-ETSU ; 2-hydroxyalkylsulfonate ; 2-hydroxyalkylthiosulfate tels que les produits commercialisés par la société GOLDSCHMIDT sous les dénominations ABIL® S201 ET ABIL® S255 ;
- des groupements hydroxyacrylamino, comme les polyorganosiloxanes décrits dans la demande EP 342 834. On peut citer, par exemple, le produit Q2-8413 de la société DOW CORNING.

Les silicones telles que décrites ci-dessus peuvent être utilisées seules ou en mélange, en une quantité comprise entre 0,01 et 20% en poids, de préférence entre 0,1 et 5% en poids.

Les compositions de l'invention peuvent encore comporter des corps gras tels que les huiles minérales, végétales, animales et synthétiques, les cires, les esters gras, les alcools gras, et les acides gras.

Comme huiles utilisables dans la composition de l'invention, on peut citer par exemple :

- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol® 810, 812 et 818 par la société Dynamit Nobel, l'huile de jojoba, l'huile de beurre de karité ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que Parléam® ; les isoparaffines comme l'isohexadécane et l'isodécane.
- les huiles fluorées partiellement hydrocarbonées et/ou siliconées comme celles décrites dans le document JP-A-2-295912 ; comme huiles fluorées, on peut citer aussi le perfluorométhylcyclopentane et le perfluoro-1,3 diméthylcyclohexane, vendus sous les dénominations de "FLUTEC® PC1" et "FLUTEC® PC3" par la Société BNFL Fluorochemicals ; le perfluoro-1,2-diméthylcyclobutane ; les perfluoroalcanes tels que le dodécafluoropentane et le tétradécafluorohexane, vendus sous les dénominations de "PF 5050®" et "PF 5060®" par la Société 3M, ou encore le bromoperfluorooctyle vendu sous la dénomination "FORALKYL®" par la Société Atochem ; le nonafluoro-méthoxybutane et le nonafluoroéthoxyisobutane ; les dérivés de perfluoromorpholine, tels que la 4-trifluorométhyl perfluoromorpholine vendue sous la dénomination "PF 5052®" par la Société 3M ;

La cire ou les cires sont choisies notamment, parmi la cire de Carnauba, la cire de Candelila, et la cire d'Alfa, la cire de paraffine, l'ozokérite, les cires végétales comme la cire d'olivier, la cire de riz, la cire de jojoba hydrogénée ou les cires absolues de fleurs telles que la cire essentielle de fleur de cassis vendue par la société BERTIN (France), les cires animales comme les cires d'abeilles, ou les cires d'abeilles modifiées (cerabellina) ; d'autres cires ou matières premières cireuses utilisables selon l'invention sont notamment les cires marines telles que celle vendue par la Société SOPHIM sous la référence M82, les cires de polyéthylène ou de polyoléfines en général.

Les acides gras saturés ou insaturés sont choisis plus particulièrement parmi l'acide myristique, l'acide palmitique, l'acide stéarique, l'acide béhénique, l'acide oléïque, l'acide linoléïque, l'acide linolénique et l'acide isostéarique.

Les esters gras sont notamment les esters d'acides carboxyliques en particulier les esters mono, di, tri ou tétracarboxyliques.

Les esters d'acides carboxyliques sont notamment les esters d'acides aliphatiques saturés ou insaturés, linéaires ou ramifiés en $C_1$-$C_{26}$ et d'alcools aliphatiques saturés ou insaturés, linéaires ou ramifiés en $C_1$-$C_{26}$, le nombre total de carbone des esters étant supérieur ou égal à 10.

Parmi les monoesters, on peut citer le béhénate de dihydroabiétyle ; le béhénate d'octyldodécyle ; le béhénate d'isocétyle ; le lactate de cétyle ; le lactate d'alkyle en $C_{12}$-$C_{15}$ ; le lactate d'isostéaryle ; le lactate de lauryle ; le lactate de linoléyle ; le lactate d'oléyle ; l'octanoate de (iso)stéaryle ; l'octanoate d'isocétyle ; l'octanoate d'octyle ; l'octanoate de cétyle ; l'oléate de décyle ; l'isostéarate d'isocétyle ; le laurate d'isocétyle ; le stéarate d'isocétyle ; l'octanoate

d'isodécyle ; l'oléate d'isodécyle ; l'isononanoate d'isononyle ; le palmitate d'isostéaryle ; le ricinoléate de méthyle acétyle ; le stéarate de myristyle ; l'isononanoate d'octyle ; l'isononate de 2-éthylhexyle ; le palmitate d'octyle ; le pélargonate d'octyle ; le stéarate d'octyle ; l'érucate d'octyldodécyle ; l'érucate d'oléyle ; les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle.

On peut également utiliser les esters d'acides di ou tricarboxyliques en $C_4$-$C_{22}$ et d'alcools en $C_1$-$C_{22}$ et les esters d'acides mono di ou tricarboxyliques et d'alcools di, tri, tétra ou pentahydroxy en $C_2$-$C_{26}$. On peut notamment citer : le sébacate de diéthyle ; le sébacate de diisopropyle ; l'adipate de diisopropyle ; l'adipate de di n-propyle ; l'adipate de dioctyle ; l'adipate de diisostéaryle ; le maléate de dioctyle ; l'undecylénate de glycéryle ; le stéarate d'octyldodécyl stéaroyl ; le monoricinoléate de pentaérythrityle ; le tétraisononanoate de pentaérythrityle ; le tétrapélargonate de pentaérythrityle ; le tétraisostéarate de pentaérythrityle ; le tétraoctanoate de pentaérythrityle ; le dicaprylate de propylène glycol ; le dicaprate de propylène glycol ; l'érucate de tridécyle ; le citrate de triisopropyle ; le citrate de triisotéaryle ; trilactate de glycéryle ; trioctanoate de glycéryle ; le citrate de trioctyldodécyle ; le citrate de trioléyle ; le dioctanoate de propylène glycol et le diheptanoate de néopentyl glycol. Les esters cités ci-dessus étant différents des esters de formule (I).

Parmi les esters cités ci-dessus, on préfère utiliser les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle et l'isononanate d'isononyle, l'octanoate de cétyle.

Comme alcools gras on peut citer les alcools gras saturés ou insaturés, linéaires ou ramifiés ayant de 8 à 26 atomes de carbone, comme l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique.

Les corps gras représentent en général de 0,1 à 50%; de préférence 1 à 30%, et encore plus préférentiellement de 2 à 20% en poids de la composition totale.

L'homme du métier saurait ajouter les additifs sans perturber les propriétés des compositions de l'invention.

Les compositions selon l'invention peuvent se présenter sous toutes les formes appropriées pour une application topique, notamment sous forme de solutions du type lotion ou sérum ; sous forme de gels aqueux ; sous forme d'émulsions obtenues par dispersion d'une phase grasse dans une phase aqueuse (H/E) ou inversement (E/H), de consistance liquide plus ou moins épaisse telle que des laits et des crèmes plus ou moins onctueuses ; sous forme de mousse, sous forme de spray, ou d'aérosol ; ou encore sous forme de brosse ou de stick.

Lorsque la composition selon l'invention est conditionnée dans un dispositif aérosol, elle comprend au moins un agent propulseur, qui peut être choisi parmi les hydrocarbures volatiles, tels que le N-butane, le propane, l'isobutane, le pentane, les hydrocarbures halogénés et leurs mélanges. On peut également utiliser en tant que propulseur le gaz carbonique, le protoxyde d'azote, le diméthyléther (DME), l'azote, ou l'air comprimé. On peut aussi utiliser des mélanges de propulseurs. De préférence, on utilise le diméthyl éther.

Avantageusement, l'agent propulseur est présent à une concentration comprise entre 5 et 90% en poids par rapport au poids total de la composition dans le dispositif aérosol, et, plus particulièrement, à une concentration comprise entre 10 et 60%.

La composition selon l'invention peut notamment être utilisée en application non rincée sur les cheveux.

L'invention a également pour objet un procédé de traitement des matières kératiniques comprenant l'application d'une composition cosmétique selon l'invention, et un procédé de mise en forme des cheveux, comprenant l'application d'une composition cosmétique selon l'invention.

Les exemples suivants illustrent la présente invention.

**Exemple 1 :** <u>Gel de coiffage selon l'invention</u>

La composition présente la formulation donnée dans le tableau 1 suivant (en poids par rapport au poids total de la composition) :

**Tableau 1**

| | |
|---|---|
| Polyuréthane cationique à séquence polyoléfine(1) | 6% |
| Polyéthyleneglycol 6000 Distéarate(2) | 3% |
| Simulsol 220 TM(3) | 2% |
| Propylène glycol | 2,5% |

(suite)

| | |
|---|---|
| Jaguar HP 105[4] | 1 % |
| Eau déminéralisée | QSP 100% |

[1] polyuréthane (A) en dispersion aqueuse formé à partir de 8,7% de N-méthyl diéthanolamine, de 23,4% d'isophorone diisocyanate, de 67,9% de KRASOL LBH2000 (polybutadiène à fonctions terminales hydroxyles) et neutralisé à 40% par HCL.
[2] Distéarate de polyéthylène glycol commercialisé par la société AKZO NOBEL. (PEG150 DISTEARATE)
[3] Mono-stéarate de glycéryle oxyéthylène commercialisé par la société SEPPIC.
[4] Hydroxypropyl guar, commercialisé par la société RHODIA.

Appliqué sur les cheveux, ce gel leur assure un coiffage avec une très longue tenue dans le temps, résistant bien à l'humidité, à l'eau et aux contraintes mécaniques. Ce gel asseure également une bonne élimination du collant des mains, et une bonne élimination au shampooing.

Ce gel, appliqué sur cheveux africains, permet d'obtenir des coiffures plaquées, très longue tenue, avec un toucher cosmétique, notamment de la douceur, après l'élimination au shampooing. Là aussi, une bonne résistance à l'humidité, à l'eau et aux contraintes mécaniques est observée.

Un résultat similaire est obtenu en utilisant un polyuréthane (B) en dispersion aqueuse formé à partir de 8,4% de poly(tetraméthylène oxyde), 8,6% de N-méthyl diéthanolamine, de 21,4% d'isophorone diisocyanate, de 61,6% de KRATON L2203 polybutadiène à fonctions terminales hydroxyles) et neutralisé à 40% par HCL.

## Revendications

**1.** Composition cosmétique comprenant, dans un milieu cosmétiquement acceptable :

(i) au moins un polyuréthane cationique comportant au moins un motif non ionique dérivé d'un homo- et/ou copolymère d'oléfine, et
(ii) au moins un ester de polyéthylène glycol.

**2.** Composition cosmétique selon la revendication 1, **caractérisée en ce que** 50% en poids ou plus des motifs non ioniques du polyuréthane dérivent d'un homopolymère ou copolymère d'oléfine.

**3.** Composition cosmétique selon la revendication 1, **caractérisée en ce que** la totalité des motifs non ioniques du polyuréthane dérivent d'un homopolymère ou copolymère d'oléfine.

**4.** Composition cosmétique selon l'une quelconque des revendications 1 à 3, **caractérisée en ce** les homopolymères et copolymères d'oléfines sont des homopolymères et copolymères portant à leurs extrémités des fonctions à hydrogène labile, et présentant des motifs choisis parmi les motifs d'éthylène, de propylène, de 1-butylène, de 2-butylène, d'isobutylène, de 1,2-butadiène, de 1,4-butadiène, d'isoprène et leurs mélanges.

**5.** Composition cosmétique selon la revendication 4 **caractérisée en ce que** les homopolymères et copolymères d'oléfines sont issus de 1,2 et/ou 1,4 butadiène éventuellement hydrogénés.

**6.** Composition cosmétique selon l'une quelconque des revendications précédentes **caractérisée en ce que** le polyuréthane cationique comprend:

(a) des motifs cationiques résultant de la réaction d'au moins une amine tertiaire ou quaternaire présentant au moins deux fonctions réactives à hydrogène labile,
(b) des motifs non ioniques dont au moins un motif (b1) résulte de la réaction d'au moins un polymère choisi parmi les homopolymères et les copolymères d'oléfines portant à leurs extrémités des fonctions réactives à hydrogène labile et ayant une température de transition vitreuse (Tg), mesurée par analyse enthalpique différentielle, inférieure à 10°C, et
(c) des motifs résultant de la réaction d'au moins un diisocyanate.

7. Composition cosmétique selon la revendication 6, **caractérisée en ce que** les motifs cationiques (a) résultent de la réaction d'au moins une amine tertiaire ou quaternaire présentant deux fonctions réactives à hydrogène labile.

8. Composition cosmétique selon la revendication 7, **caractérisée en ce que** l'amine est choisie parmi les amines de formules suivantes :

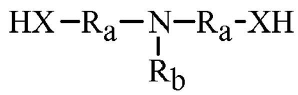
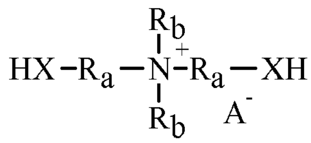
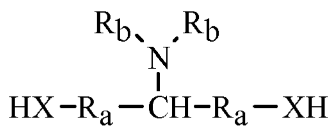
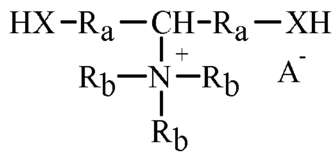
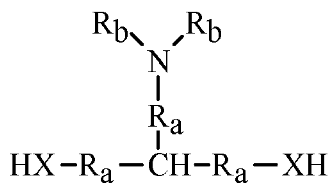
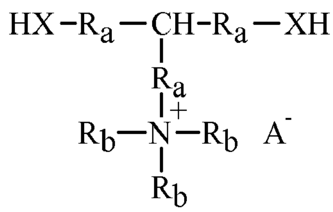

dans lesquelles

chaque $R_a$ représente indépendamment un groupe alkylène en $C_1$-$C_6$, linéaire ou ramifié, cycloalkylène en $C_3$-$C_6$ ou arylène, ou leurs mélanges ; tous pouvant être substitués par un ou plusieurs atomes d'halogène et comporter un ou plusieurs hétéroatomes choisis parmi O, N, P et S,
chaque $R_b$ représente indépendamment un groupe alkyle en $C_1$-$C_6$, cycloalkyle en $C_3$-$C_6$ ou aryle, ou leurs mélanges ; tous pouvant être substitués par un ou plusieurs atomes d'halogène et comporter un ou plusieurs atomes d'halogène et comporter un ou plusieurs hétéroatomes choisis parmi O, N, P et S,
chaque X représente indépendamment un atome d'oxygène ou de soufre ou un groupe NH ou $NR_c$, où $R_c$ représente un groupe alkyle en $C_1$-$C_6$, et
$A^-$ représente un contre-ion physiologiquement acceptable.

9. Composition cosmétique selon la revendication 8, **caractérisée en ce que** les motifs cationiques (a) résultent de la réaction de la N-méthyldiéthanolamine ou de la N-tert-butyldiéthanolamine.

10. Composition cosmétique selon la revendication 7, **caractérisée en ce que** les motifs (a) résultent de la réaction d'au moins un polymère à fonction(s) amine tertiaire et/ou quaternaire, portant à ses extrémités des fonctions réactives à hydrogène labile choisies parmi -OH, $-NH_2$, $-NHR_c$ ou -SH, et ayant une masse moléculaire en poids comprise entre 400 et 10 000, $R_c$ représentant un groupe alkyle en $C_1$-$C_6$.

11. Composition cosmétique selon l'une quelconque des revendications 6 à 10, **caractérisée en ce que** le polyuréthane cationique contient éventuellement au moins un motif non ionique (b2), différent du motif (b1), dérivant d'un composé non ionique monomère contenant au moins deux fonctions à hydrogène labile capables de réagir avec le ou lesdits composés (c) contenant au moins un diisocyanate.

**12.** Composition cosmétique selon la revendication 11, **caractérisée en ce que** les motifs cationiques (a) représentent de 0,1 à 90%, de préférence de 1 à 30%, mieux de 1 à 20% en poids, les motifs non-ioniques dérivés d'un homo- ou copolymère d'oléfine (b1) représentent de 10 à 99,9%, de préférence de 20 à 99% et mieux de 30 à 85% en poids, et les motifs non-ioniques (b2) représentent de 0 à 50%, de préférence 0 à 30% en poids du total des motifs du polyuréthane cationique.

**13.** Composition cosmétique selon l'une quelconque des revendications 6 à 11, **caractérisée en ce que** le diisocyanate est choisi parmi le méthylènediphényldiisocyanate, le méthylènecyclohexanediisocyanate, l'isophoronediisocyanate, le toluènediisocyanate, le naphtalènediisocyanate, le 1,4-butanediisocyanate et le 1,6-hexane-diisocyanate.

**14.** Composition cosmétique selon la revendication 13, **caractérisée en ce que** le diisocyanate est l'isophoronediisocyanate.

**15.** Composition cosmétique selon l'une quelconque des revendications 6 à 14, **caractérisée en ce que** les motifs (c) représentent de 1 à 60%, de préférence de 5 à 50%, et idéalement 1 à 40% du poids total des motifs du polyuréthane cationique.

**16.** Composition cosmétique selon l'une quelconque des revendications 6 à 15, **caractérisée en ce que** le ou les composés non ioniques monomères formant les motifs non ioniques (b2) sont choisis parmi les diols en $C_1$-$C_{12}$, de préférence le néopentylglycol, l'hexa(éthylèneglycol), le 1,2-éthanediol, le 1,2-propanediol et le 1,3-propanediol, et les aminoalcools en $C_1$-$C_6$, de préférence l'aminoéthanol.

**17.** Composition cosmétique selon l'une quelconque des revendications 6 à 16, **caractérisée en ce que** le polyuréthane cationique ne comprend pas de motifs en plus des motifs (a), (b) et (c).

**18.** Composition cosmétique selon l'une quelconque des revendications 6 à 17, **caractérisée en ce que** le polyuréthane cationique présente un caractère élastique.

**19.** Composition selon l'une quelconque des revendications précédentes, **caractérisée en ce que** le (ou les) polyuréthane(s) cationique(s) représente(nt) de 0,01% à 40%, de préférence 0,05 à 20% et idéalement de 0,1 à 10% en poids rapporté à la composition cosmétique finale.

**20.** Composition cosmétique selon l'une quelconque des revendications précédentes, **caractérisée en ce que** l'ester de polyéthylène glycol est défini par la formule suivante :

$$X\text{-}(OCH_2CH_2)_mOY \qquad (I)$$

- m allant de 80 à 500 et
- X et Y représentant, indépendemment l'un de l'autre un atome d'hydrogène ou un groupement acyle en $C_8$ à $C_{30}$ linéaire ou ramifié, saturé ou insaturé,

à condition que l'un au moins des groupements X et Y représente un groupement acyle.

**21.** Composition cosmétique selon la revendication 20, **caractérisée en ce que** l'ester de polyéthylène glycol est un monoester.

**22.** Composition cosmétique selon la revendication 20, **caractérisée en ce que** l'ester de polyéthylène glycol est un diester.

**23.** Composition cosmétique selon la revendication 20, **caractérisée en ce que** m va de 80 à 350, et encore plus préférentiellement de 100 à 300.

**24.** Composition cosmétique selon la revendication 20, **caractérisée en ce que** le rapport R - masse de la partie hydrophyle $(OCH_2\ CH_2)_m$/(masse de l'ester de polyéthylèneglycol-masse de la partie hydrophyle $(OCH_2\ CH_2)_m$) est compris entre 8 et 1000.

**25.** Composition cosmétique selon l'une quelconque des revendications précédentes, **caractérisée en ce qu'**elle comprend entre 0,01 et 50 % en poids, de préférence entre 0,1 et 20 %, encore plus préférentiellement entre 0,5 et

15% en poids, d'ester de polyéthylène glycol, rapporté au poids de la composition cosmétique finale.

**26.** Composition selon l'une quelconque des revendications précédentes, **caractérisée en ce qu'**elle comprend un ou plusieurs additifs choisis parmi les agents gélifiants et/ou épaississants, les agents tensioactifs, les silicones, les solvants organiques, les parfums, les huiles minérales, végétales et/ou synthétiques, les esters d'acide gras, les agents de stabilisation de pH, les agents conservateurs et les agents absorbants les UV.

**27.** Composition selon l'une quelconque des revendications précédentes, **caractérisée en ce qu'**elle contient au moins un agent propulseur et est conditionnée sous forme d'un aérosol.

**28.** Procédé de coiffage comprenant l'application d'une composition selon l'une quelconque des revendications 1 à 26 sur les cheveux, le rinçage éventuel des cheveux, puis la mise en forme et le séchage des cheveux.

Office européen des brevets

# RAPPORT DE RECHERCHE EUROPEENNE

Numéro de la demande
EP 08 30 0018

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | DATABASE WPI Week 197903<br>Derwent Publications Ltd., London, GB; AN 1979-04952B<br>XP002453389<br>& JP 53 139735 A (DAIICHI KOGYO SEIYAKU CO LTD) 6 décembre 1978 (1978-12-06)<br>* abrégé * | 1-28 | INV.<br>A61K8/86<br>A61K8/87<br>A61Q5/06 |
| D,Y | FR 2 815 350 A1 (OREAL [FR])<br>19 avril 2002 (2002-04-19)<br>* page 3, ligne 5-9,25 - page 7, ligne 23; revendications 1-19; exemples 1,2 * | 1-28 | |
| Y | EP 1 645 579 A (OREAL [FR])<br>12 avril 2006 (2006-04-12)<br>* alinéas [0008], [0012], [0013], [0016], [0020], [0023], [0036], [0053]; revendications 1-32; exemples 2-4 * | 1-28 | |
| D,Y | FR 2 833 960 A1 (OREAL [FR])<br>27 juin 2003 (2003-06-27)<br>* page 1, ligne 23-28 *<br>* page 2, ligne 27-32 *<br>* page 3, ligne 19 - page 6, ligne 23; revendications 1-26; exemple 1 * | 1-28 | DOMAINES TECHNIQUES RECHERCHES (IPC)<br>A61K |

Le présent rapport a été établi pour toutes les revendications

| Lieu de la recherche | Date d'achèvement de la recherche | Examinateur |
|---|---|---|
| Munich | 25 mars 2008 | Yon, Jean-Michel |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

2

EPO FORM 1503 03.82 (P04C02)

**ANNEXE AU RAPPORT DE RECHERCHE EUROPEENNE RELATIF A LA DEMANDE DE BREVET EUROPEEN NO.** EP 08 30 0018

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

25-03-2008

| Document brevet cité au rapport de recherche | | Date de publication | Membre(s) de la famille de brevet(s) | | | Date de publication |
|---|---|---|---|---|---|---|
| JP 53139735 | A | 06-12-1978 | AUCUN | | | |
| FR 2815350 | A1 | 19-04-2002 | AR | 030894 | A1 | 03-09-2003 |
| | | | AU | 9569201 | A | 29-04-2002 |
| | | | EP | 1326908 | A1 | 16-07-2003 |
| | | | WO | 0232978 | A1 | 25-04-2002 |
| | | | JP | 2004511637 | T | 15-04-2004 |
| | | | US | 2004052753 | A1 | 18-03-2004 |
| | | | US | 2008025933 | A1 | 31-01-2008 |
| EP 1645579 | A | 12-04-2006 | BR | PI0506007 | A | 06-06-2006 |
| | | | CN | 1775826 | A | 24-05-2006 |
| | | | FR | 2875503 | A1 | 24-03-2006 |
| | | | JP | 2006104463 | A | 20-04-2006 |
| | | | KR | 20060051504 | A | 19-05-2006 |
| FR 2833960 | A1 | 27-06-2003 | EP | 1323756 | A1 | 02-07-2003 |
| | | | JP | 2003226733 | A | 12-08-2003 |
| | | | US | 2004001798 | A1 | 01-01-2004 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9413724 A
- EP 0619111 A
- FR 2815350
- FR 2833960
- US 2528378 A
- US 2781354 A
- FR 8516334 A
- US 4957732 A
- EP 186507 A
- EP 342834 A
- JP 2295912 A


### Littérature non-brevet citée dans la description


- International Cosmetic Ingredient Dictionary and Handbook. 2002
- **M.R. PORTER.** Handbook of Surfactants. Blackie & Son, 1991, 116-178
- **WALTER NOLL.** Chemistry and Technology of Silicones. Academie Press, 1968
- **TODD ; BYERS.** Volatile Silicone fluids for cosmetics. *Cosmetics and Toiletries,* Janvier 1976, vol. 91, 27-32